# EUROPEAN PATENT APPLICATION

(11) **EP 3 996 036 A1**
(43) Date of publication of application: **11.05.2022**
(21) Application number: 20206429.1
(22) Date of filing: 09.11.2020
(51) Int. Cl.: G06T 7/00, A61B 5/055, G06T 7/11

(54) **MAGNETIC RESONANCE ANGIOGRAPHY USING T2 WEIGHTED IMAGES**

(71) Applicant: Koninklijke Philips N.V., 5656 AG Eindhoven (NL)
(72) Inventor: LAMERICHS, Rudolf Mathias Johannes Nicolaas, 5656 AE Eindhoven (NL); SU, Jiahang, 5656 AE Eindhoven (NL)
(74) Representative: Philips Intellectual Property & Standards

(57) **Abstract**

Disclosed herein is a medical system (100, 300) comprising a memory (110) storing machine executable instructions (120) and a computational system (104), wherein execution of the machine executable instructions causes the computational system to: receive (200) dark blood T2 weighted magnetic resonance imaging data (122) descriptive of a brain region of a subject; and segment (202) the dark blood T2 weighted magnetic resonance imaging data for blood vessels to provide a magnetic resonance angiogram (124) of the brain region.

## Description

### FIELD OF THE INVENTION

The invention relates to magnetic resonance imaging, in particular to magnetic resonance angiography.

### BACKGROUND OF THE INVENTION

A large static magnetic field is used by Magnetic Resonance Imaging (MRI) scanners to align the nuclear spins of atoms as part of the procedure for producing images within the body of a patient. This large static magnetic field is referred to as the B0 field or the main magnetic field. Various quantities or properties of the subject can be measured spatially using MRI. One magnetic resonance imaging technique is magnetic resonance angiography. Magnetic resonance angiography includes a variety of different magnetic resonance imaging techniques that are used to map the location of the vascular system of a subj ect.

United States patent application US 2012099779 A1 discloses a method for segmenting a brain image into a CSF region, a WM region and a GM region. An upper limit for the intensity values of a CSF region in the image is estimated such that the points of the image having an intensity less than this upper limit include a subset of the points which form a spatially connected group and which have a peaked intensity distribution. In other words, the invention exploits both the expected spatial distribution and expected intensity distribution of the CSF region. This makes it possible for the method to provide reliable discrimination of the CSF region even in CT images with poor image quality. Various methods are proposed for using the upper limit, and for improving the segmentation accuracy.

### SUMMARY OF THE IVENTION

The invention provides for a medical system, a method, and a computer program in the independent claims. Embodiments are given in the dependent claims.

Magnetic resonance angiography techniques typically rely on either injecting the subject with a contrast agent or mapping the flow of blood in a subject. When examining MR brain angiograms physician would like to have a mapping of not only of the blood vessels and arteries in a subject but also see how they relate to the anatomy of a subject. To do this, separate MR angiograms and detailed magnetic resonance images are acquired. A disadvantage of this is that is very time consuming, the subject may move during the multiple acquisitions, and the MR angiogram needs to be registered to the detailed magnetic resonance image. Embodiments may solve these problems by producing a magnetic resonance angiogram from a segmentation of a dark blood T2 weighted magnetic resonance imaging data. A potential advantage is that the overall acquisition time may be greatly reduced. Another potential advantage may be that since the magnetic resonance angiogram was produced using a segmentation of the dark blood T2 weighted magnetic resonance imaging data is that the angiogram is automatically positioned correctly in the anatomy of the dark blood T2 weighted magnetic resonance imaging data.

In one aspect the invention provides for a medical system that comprises a memory storing machine-executable instructions and a computational system. Execution of the machine-executable instructions causes the computational system to receive dark blood T2 weighted magnetic resonance imaging data descriptive of a brain region of a subject. Execution of the machine-executable instructions further causes the computational system to segment the dark blood T2 weighted magnetic resonance imaging data for blood vessels to provide a magnetic resonance angiogram of the brain region.

A dark blood T2 weighted magnetic resonance imaging data is magnetic resonance imaging data that has been acquired using a pulse sequence that acquires T2 weighted data to be acquired and also such that blood has a dark or low contrast. The providing of the magnetic resonance angiogram using the dark blood T2 weighted magnetic resonance imaging data may be beneficial because it eliminates the need to acquire separate angiogram data. In addition, since the angiogram was derived from the dark blood T2 weighted magnetic resonance imaging data it is automatically aligned with the anatomical structures shown in the dark blood T2 weighted magnetic resonance imaging data. There is therefore no need to register the angiogram to any T2 weighted magnetic resonance imaging data. This may for example, be a particular benefit in planning radiotherapy and invasive procedures on the brain of the subject.

In another embodiment, the brain region is a whole brain region.

In another embodiment execution of the machine-executable instructions further causes the computational system to segment the blood vessels in the dark blood T2 weighted magnetic resonance imaging data with the steps comprising of obtaining a binary brain mask for the dark blood T2 weighted magnetic resonance imaging data by inputting the dark blood T2 weighted magnetic resonance imaging data into a brain segmentation algorithm. A variety of segmentation algorithms are available for segmenting the cranial or brain of a subject. The brain binary mask is a binary mask which indicates the location of the brain of the subject in the dark blood T2 weighted magnetic resonance imaging data. The steps further comprise obtaining a brain tissue image by multiplying the dark blood T2 weighted magnetic resonance imaging data with the binary brain mask. The effect of this multiplication is that tissue outside of the brain of the subject are effectively eliminated.

The steps further comprise providing a blood vessel segmentation by segmenting the brain tissue image for blood vessels. This is possible because in the dark blood T2 weighted magnetic resonance imaging data the blood vessels are dark. Black or dark structures within the brain tissue image will be the blood vessels. The steps further comprise providing the blood vessel segmentation as the magnetic resonance angiogram. The detail of the magnetic resonance angiogram can be chosen by choosing the resolution of the dark blood T2 weighted magnetic resonance imaging data.

In another embodiment execution of the machine-executable instructions further causes the computational system to provide the blood vessel segmentation by summing blood vessel segmentations and multiple images derived from the brain tissue image. This embodiment may be beneficial because the brain tissue image can be processed using a variety of algorithms and segmentation steps. By using multiple segmentations, the entire vascularization of the brain tissue can be determined.

In another embodiment the segmentation of the dark blood T2 weighted magnetic resonance imaging data comprises a cerebrospinal fluid region segmentation. Execution of the machine-executable instructions further causes the computational system to calculate an inverted brain tissue image by inverting the brain tissue image. Execution of the machine-executable instructions further causes the computational system to calculate an inverted cerebrospinal fluid region using the cerebrospinal fluid region segmentation and an inversion of the brain tissue image.

The multiple images derived from the brain tissue image comprise the inverted cerebrospinal fluid region. Within the cerebrospinal fluid region, the regions with the blood vessels have a dark appearance and the cerebrospinal fluid is bright. Upon inversion, the blood vessel and vascularization become bright and the cerebrospinal fluid becomes dark. This makes it easy to segment the tissue to determine the position of the blood vessels. This, for example, may be done using a more complicated segmentation algorithm or it may be done simply using a thresholding. In this embodiment the cerebrospinal fluid region may be part of the brain tissue image.

In another embodiment execution of the machine-executable instructions further causes the computational system to calculate a diffusion filtered brain tissue image by applying a three-dimensional diffusion filter to the inverted brain tissue image. Execution of the machine-executable instructions further causes the computational system to calculate a contrast enhanced brain tissue image by applying a contrast enhancement filter to the diffusion filtered brain tissue image. The multiple images derived from the brain tissue image comprise the contrast enhanced brain tissue image. Execution of the machine-executable instructions further causes the computational system to calculate a Frangi filtered brain tissue image by applying a Frangi filter to the diffusion filtered brain tissue image. The multiple images derived from the brain tissue image comprise the Frangi filtered brain tissue image.

In another embodiment the segmentation of the multiple images is performed using a gradient-based segmentation. The use of a gradient-based segmentation may for example be more effective than using a thresholding segmentation because the contrast of the magnetic resonance image may not be uniform across its entire field of view.

In another embodiment execution of the machine-executable instructions further causes the computational system to construct the magnetic resonance angiogram by applying a gap-filling algorithm to the combined segmentations of multiple images. The multiple images are combined to provide the angiogram; however there may be gaps between them. The gap-filling algorithm may be used to bridge these gaps and provide a complete angiogram.

In another embodiment execution of the machine-executable instructions further causes the computational system to identify arterial and venous flow in the magnetic resonance angiogram. This may for example be performed by identifying the veins and arteries in the angiogram and marking these. For example, any region which is identified as being part of the vascular structure of the brain would have some either arterial or venous flow.

In another embodiment execution of the machine-executable instructions further causes the computational system to render the dark blood T2 weighted magnetic resonance imaging data and the magnetic resonance angiogram three-dimensionally to provide a therapy planning user interface. The magnetic resonance angiogram is superimposed on the dark blood T2 weighted magnetic resonance imaging data during the rendering. The therapy planning system is further configured to render a therapy path in the therapy planning user interface. This embodiment may be beneficial because the dark blood T2 weighted magnetic resonance imaging data has been segmented and also may be used to display the anatomy of the subject's brain. Because the magnetic resonance angiogram was derived from the dark blood T2 weighted magnetic resonance imaging data the anatomy of these two medical images coincide perfectly with each other without the need for alignment. This may for example, provide more accurate determination of the therapy path.

In another embodiment the therapy path is a radiotherapy path. For example, this may be the path that a radiation beam is used to treat a target region.

In another embodiment the therapy path is a biopsy needle insertion path.

In another embodiment the therapy path is a deep brain stimulation electrode insertion path.

In another embodiment the therapy path is a surgical path.

In another embodiment the medical system further comprises a magnetic resonance imaging system controlled by the computational system. The memory further stores pulse sequence commands configured for acquiring k-space data according to a dark blood T2 weighted magnetic resonance imaging protocol. Execution of the machine-executable instructions further causes the computational system to acquire the k-space data by controlling the magnetic resonance imaging system with the pulse sequence commands. Execution of the machine-executable instructions further causes the computational system to reconstruct the dark blood T2 weighted magnetic resonance imaging data from the k-space data. This embodiment may be beneficial because both the dark blood T2 weighted magnetic resonance imaging data and the magnetic resonance angiogram are derived from the same k-space data. They therefore automatically have their anatomical structures aligned and may for example provide for better guidance during a surgical procedure.

In another embodiment the dark blood T2 weighted magnetic resonance imaging protocol is a three-dimensional T2 weighted turbo-spin echo magnetic resonance imaging protocol. This particular magnetic resonance imaging protocol may be beneficial because it produces a high signal from the cerebrospinal fluid in contrast to blood. This may for example, make it easier to determine the location of blood vessels and other vascularization within the brain of the subject.

In another embodiment the dark blood T2 weighted magnetic resonance imaging data has a resolution of 0.5 mm or lower. This embodiment may be beneficial because it may enable the detection of blood vessels which have a diameter of 0.5 mm or larger.

In another embodiment the dark blood T2 weighted magnetic resonance imaging data has a resolution of 0.4 mm or lower. This embodiment may also be beneficial and may enable the detection of blood vessels with a diameter of 0.4 mm and larger.

In another embodiment the dark blood T2 weighted magnetic resonance imaging data has a resolution of 0.35 mm or lower. This embodiment may be beneficial because blood vessels with a yet smaller diameter may be detected.

In another aspect the invention provides for a computer program comprising machine-executable instructions for execution by a computational system. The computer program may for example be stored on a non-transitory medium such as a memory, hard drive or other storage device. Execution of the machine-executable instructions causes the computational system to receive dark blood T2 weighted magnetic resonance imaging data descriptive of a brain region of a subject. Execution of the machine-executable instructions further causes the computational system to segment the dark blood T2 weighted magnetic resonance imaging data for blood vessels to provide a magnetic resonance angiogram of the brain region. The advantages of this embodiment have been previously discussed.

In another aspect the invention provides a method of operating a medical system. The method comprises receiving dark blood T2 weighted magnetic resonance imaging data descriptive of a brain region of a subject. The method further comprises segmenting the dark blood T2 weighted magnetic resonance imaging data for blood vessels to provide a magnetic resonance angiogram of the brain region. The advantages of this embodiment have also been discussed.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, microcode, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid-state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a computational system. 'Computer storage' or 'storage' is a further example of a computer-readable storage medium. Computer storage is any non-volatile computer-readable storage medium. In some embodiments computer storage may also be computer memory or vice versa.

A 'computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a computational system of a general purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.
A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.
A 'hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen, Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

Medical imaging data is defined herein as being recorded measurements made by a tomographic medical imaging system descriptive of a subject. The medical imaging data may be reconstructed into a medical image. A medical image id defined herein as being the reconstructed two- or three-dimensional visualization of anatomic data contained within the medical imaging data. This visualization can be performed using a computer.

K-space data is defined herein as being the recorded measurements of radio frequency signals emitted by atomic spins using the antenna of a Magnetic resonance apparatus during a magnetic resonance imaging scan. Magnetic resonance data is an example of tomographic medical image data.

Magnetic resonance imaging data or a magnetic resonance image is defined herein as being the reconstructed two- or three-dimensional visualization of anatomic data contained within the magnetic resonance imaging data. This visualization can be performed using a computer.

### BRIEF DESCRIPTION OF THE DRAWINGS

In the following preferred embodiments of the invention will be described, by way of example only, and with reference to the drawings in which:
Fig. 1 illustrates an example of a medical system;
Fig. 2 shows a flow chart which illustrates a method of using the medical system of Fig. 1;
Fig. 3 illustrates a further example of a medical system;
Fig. 4 shows a flow chart which illustrates a method of using the medical system of Fig. 3;
Fig. 5 shows an exemplary dark blood T2 weighted magnetic resonance image;
Fig. 6 shows a flow chart which illustrates an automated blood vessel segmentation algorithm,
Fig. 7 illustrates the production of a brain tissue image;
Fig. 8 shows a MICO CSF segmentation;
Fig. 9 shows exemplary inverted brain tissue image and diffusion filtered brain tissue image;
Fig. 10 illustrates an example of a vessel segmentgation algorithm,
Fig. 11 shows the output of different branches of the vessel segmentation algorithm and the combined vessel segmenation;
Fig. 12 shows the output of the vessel segmenation and the result of applying a gap filling algorithm after the vessel segmentation;
Fig. 13 shows the final output image (an MR angiogram) from the algorithm illustrated in Fig. 6;
Fig. 14 shows a further example of a dark blood T2 weighted magnetic resonance image; and
Fig. 15 shows a magnetice resonance angiogram superimposed on dark blood T2 weighted magnetic resonance image of Fig. 14.

### DETAILED DESCRIPTION OF EMBODIMENTS

Like numbered elements in these figures are either equivalent elements or perform the same function. Elements which have been discussed previously will not necessarily be discussed in later figures if the function is equivalent.

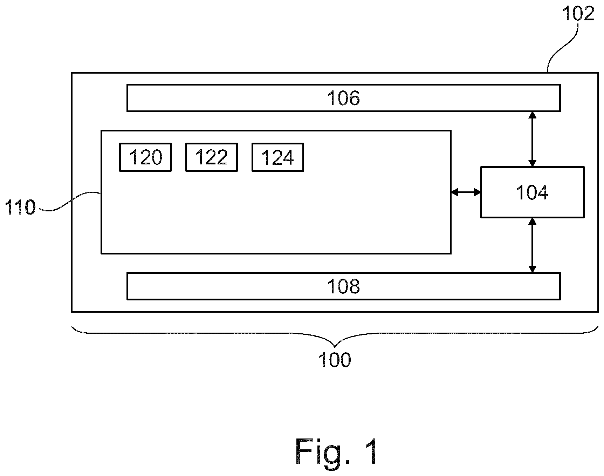

Fig. 1 illustrates an example of a medical system 100. The medical system 100 in Fig. 1 is shown as comprising a computer 102. The computer comprises a computational system 104. The computational system 104 is intended to represent one or more processor cores at one or more locations. The computational system 104 is shown as being connected to an optional hardware interface 106 and an optional user interface 108. If present, the hardware interface 106 may be used for controlling and communicating with other components of the medical system 100. If present, the user interface 108 may provide a means for an operator to interact with and control the medical system 100.

The computational system 104 is shown as further being in communication with a memory 110. The memory 110 is intended to represent any or various types of memory or storage devices which the computational system 104 may be in communication with. The memory 110 may for example be various types of volatile or non-volatile memory such as a non-transitory storage medium. The memory 110 is shown as storing machine-executable instructions 120. The machine-executable instructions 120 provide instructions which may enable the computational system 104 to perform basic data analysis and image processing tasks. The memory 110 is further shown as containing dark blood T2 weighted magnetic resonance imaging data 122. The memory 110 is further shown as containing a magnetic resonance angiogram 124 that was constructed by segmenting the dark blood T2 weighted magnetic resonance imaging data 122 for blood vessels. For example, the machine-executable instructions 120 may provide instructions that enable the computational system 104 to perform this segmentation.

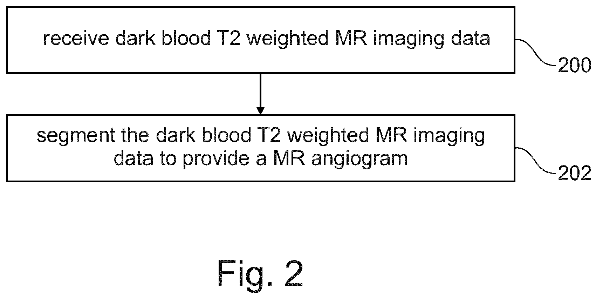

Fig. 2 shows a flowchart which illustrates a method of operating the medical system 100 of Fig. 1. First, in step 200, the dark blood T2 weighted magnetic resonance imaging data 122 is received. Next, in step 202, the dark blood T2 weighted magnetic resonance imaging data 122 is segmented to provide the magnetic resonance angiogram 124.

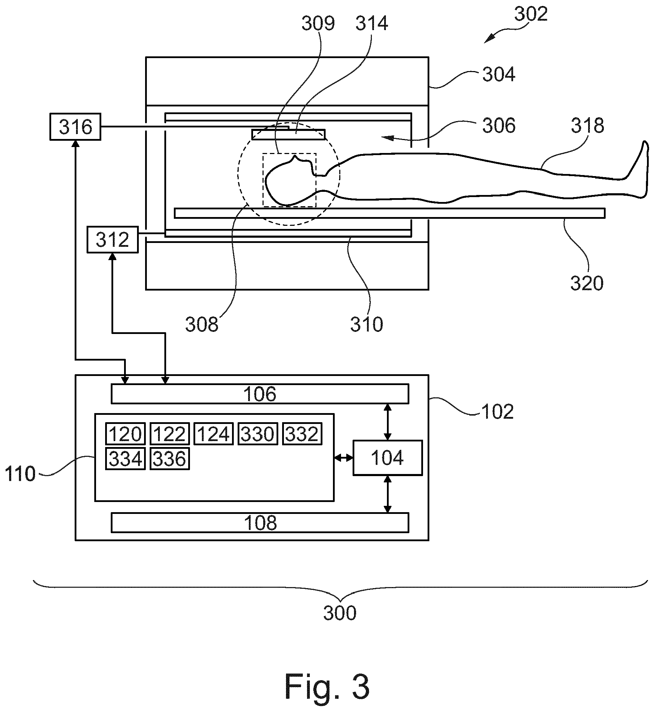

Fig. 3 illustrates a further example of a medical system 300. The medical system 300 is similar to the medical system 100 in Fig. 1 except that it additionally comprises a magnetic resonance imaging system 302 that is controlled by the computational system 104.

The magnetic resonance imaging system 302 comprises a magnet 304. The magnet 304 is a superconducting cylindrical type magnet with a bore 306 through it. The use of different types of magnets is also possible; for instance it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils.

Within the bore 306 of the cylindrical magnet 304 there is an imaging zone 308 where the magnetic field is strong and uniform enough to perform magnetic resonance imaging. A region of interest 309 is shown within the imaging zone 308. The magnetic resonance data that is acquired typically acquried for the region of interest. A subject 318 is shown as being supported by a subject support 320 such that at least a portion of the subject 318 is within the imaging zone 308 and the region of interest 309.

Within the bore 306 of the magnet there is also a set of magnetic field gradient coils 310 which is used for acquisition of preliminary magnetic resonance data to spatially encode magnetic spins within the imaging zone 308 of the magnet 304. The magnetic field gradient coils 310 connected to a magnetic field gradient coil power supply 312. The magnetic field gradient coils 310 are intended to be representative. Typically magnetic field gradient coils 310 contain three separate sets of coils for spatially encoding in three orthogonal spatial directions. A magnetic field gradient power supply supplies current to the magnetic field gradient coils. The current supplied to the magnetic field gradient coils 310 is controlled as a function of time and may be ramped or pulsed.

Adjacent to the imaging zone 308 is a radio-frequency coil 314 for manipulating the orientations of magnetic spins within the imaging zone 308 and for receiving radio transmissions from spins also within the imaging zone 308. The radio frequency antenna may contain multiple coil elements. The radio frequency antenna may also be referred to as a channel or antenna. The radio-frequency coil 314 is connected to a radio frequency transceiver 316. The radio-frequency coil 314 and radio frequency transceiver 316 may be replaced by separate transmit and receive coils and a separate transmitter and receiver. It is understood that the radio-frequency coil 314 and the radio frequency transceiver 316 are representative. The radio-frequency coil 314 is intended to also represent a dedicated transmit antenna and a dedicated receive antenna. Likewise the transceiver 316 may also represent a separate transmitter and receivers. The radio-frequency coil 314 may also have multiple receive/transmit elements and the radio frequency transceiver 316 may have multiple receive/transmit channels. For example if a parallel imaging technique such as SENSE is performed, the radio-frequency could 314 will have multiple coil elements.

The transceiver 316 and the gradient controller 312 are shown as being connected to the hardware interface 106 of the computer system 102.

The memory 110 is shown as containing pulse sequence commands 330. The pulse sequence commands are commands or data which can be converted into commands which can be used to control the magnetic resonance imaging system 302 to acquire k-space data 332. In this case, the pulse sequence commands 330 are configured to acquire the k-space data according to a dark blood T2 weighted magnetic resonance imaging protocol. A good choice would be a three-dimensional T2 weighted turbo-spin echo magnetic resonance imaging protocol. The memory 110 is further shown as containing k-space data 332 that was acquired from the region of interest 309, which includes the brain of the subject, 318. The k-space data is then reconstructed by the computational system 104 into the dark blood T2 weighted magnetic resonance imaging data 122.

The computational system 104 may then perform other various operations such as constructing a rendering 334 of the dark blood T2 weighted magnetic resonance imaging data 122 and the magnetic resonance angiogram 124 that are superimposed on each other and may also be rendered three-dimensionally. The memory 110 may also contain a therapy path 336 that has been chosen or received as input from the user interface 108 in response to the rendering 334. For example, the rendering 334 may be displayed on a display and then an operator may then choose the therapy path 336 using controls on the user interface.

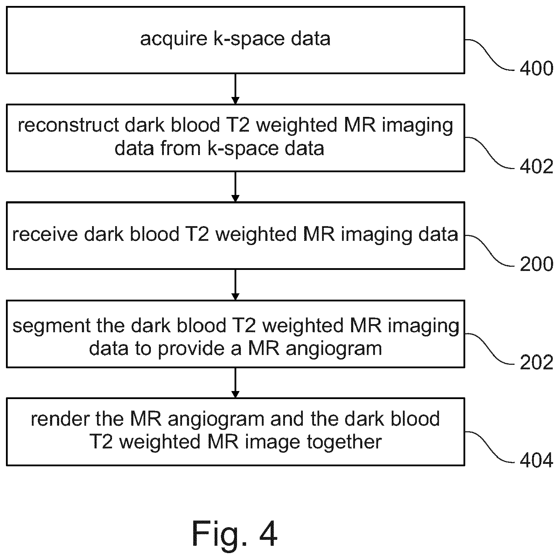

Fig. 4 shows a flowchart which illustrates a method of operating the medical system 300 of Fig. 3. First, in step 400, the magnetic resonance imaging system 302 is controlled with the pulse sequence commands 330 to acquire the k-space data 332. Next, in step 402, the dark blood t2 weighted magnetic resonance imaging data 122 is reconstructed from the k-space data 332. The method then proceeds to steps 200 and 202 of Fig. 2.

After step 202 is finished, step 404 may be optionally performed. In step 404 the dark blood t2 weighted magnetic resonance imaging data 122 and the magnetic resonance angiogram 124 are rendered together on a user interface or display. It may for example be superimposed so that the operator of the medical system 300 is able to see both the vascular structure represented in the magnetic resonance angiogram 124 as well as the anatomical structures depicted in the dark blood t2 weighted magnetic resonance imaging data 122. This for example may be very useful in choosing a therapy path 336 or planning another intervention for the subject 318.

In current clinical applications anatomical images and vessel images are recorded separately. For example, in MRI anatomical information is derived from T2 and T1 weighted images. Dedicated Magnetic Resonance Angiography (MRA) sequences are needed to visualize the vessels. In these images the stationary tissues are strongly suppressed showing predominantly the signal from the flowing tissues.

Vessel information may be used to show abnormalities like occlusions, aneurisms, AVM, but may also be used for pre-surgical planning, as in deep brain stimulations (DBS) DBS offers relief for several neurological and mental. It is mostly used in Parkinson's disease; the target is the subthalamic nucleus, a specific area located deep in the brain close to the brain-stem. DBS can also be used in patients with chronic pain or major depression, albeit with other deep brain targets. For surgical planning of the DBS procedure the exact location of the target tissue as well as the location of arteries and veins is preferably be known. This information may be used to define a save path for the needle to reach the target area in brain.

Currently MR angiograms are recorded as separate scans in clinical settings. The MRA data for the brain are mostly recorded as time-of-flight (TOF). After processing (Maximum Intensity Projection) the data may show predominantly the arterial flow in the brain. In order to see the veins a separate venogram may be recorded. Furthermore, this vessel information may be co-registered with an anatomical scan. This latter procedure is not trivial and prone to errors. Sometime the trajectory planning is also based on other modalities such CT and angiography (x-ray), which also have to be co-registered with the MR data

Examples may provide the means to extract the vessel information, for example, from a high-resolution anatomical scan acquired on, for example, 3 Tesla MRI scanners. A high-resolution T2-weighted scan of the total brain may be used for the purpose of quantifying small brain structures, such as the hippocampus and its substructures.

The resolution of this exemplary scan is 0.7 mm isotropic at acquisition and 0.35 mm at reconstruction. An additional characteristic of this scan is that vessels, i.e. arteries and veins, are black. Closer inspection of the data revealed that because of the isotropic high-resolution, the data-sets contain very detailed vessel information and that it can be extracted from the data using dedicated processing tools.

Extracting the vessel information from the high-resolution T2-weighted scan may therefore provide one or more of the following benefits: 1) result in a significant scan time reduction. Only one 7-minute scan is required; additional separate MRA scans would take another 6-10 minutes. 2) Arterial and venous flow is available from one data-set. With post-processing they can be distinguished. 3) The vessel information and anatomical target area are based on the same scan and may be combined in a 3D view for the surgical planning.

In summary, examples may provide a means for improving the surgical planning by avoiding addition processing steps, such as co-registration of images, with a significant saving of scan-time.

In some examples, information is extracted from the high-resolution T2-weighted scan and this angiogram is used in pre-surgical planning for DBS. Compared to the current approach there may be several advantages:
The most important advantage is that vessel information and the anatomical target area are based on the same scan. In the current procedure the anatomical scans and the angiograms are recorded separately and have to be co-registered, this is difficult and prone to errors. Also motion in between the various scans can lead to unacceptable errors in the co-registration.

The anatomical scan proposed in examples may have an unprecedented high-resolution, enabling a more detailed analysis of the target region. The high resolution may also allow the detection of small vessel down to 0.4 mm in diameter.

The anatomical target from the original T2-weighted scan and the angiogram can be combined in one 3D view for the surgical planning; since both are based the same scan this may be more accurate than previous visualizations, i.e. no co-registration of separately recorded data-sets.

A significant reduction of the total examination time may be a benefit also. The high-resolution scan time may be sufficient to provide all relevant information. Only one 7-minute scan is required in some examples; additional separate MRA scans could potentially take another 6-10 minutes, in the current approach, examination times of 40 minutes are not uncommon. Including survey scan and patient handling the procedure proposed here could take around 15 minutes. This shorter examination time could possibly reduce the risk of motion significantly.

Arterial and venous flow may be available from one data-set. With post-processing they can be distinguished.

The potential problems related to separate MRA scans may potentially be eliminated. For example, some of the pitfalls of TOF-MRA are slow flow or flow from a vessel parallel to the scan-plane, may become de-saturated just like stationary tissue, resulting in signal loss from the vessel turbulent flow may undergo spin-dephasing and unexpectedly short T2 relaxation: again resulting in signal loss from the vessel acquisition times are relatively long. retrograde arterial flow may be obscured if venous saturation bands have been applied.

In summary, examples may provide a means for improving the surgical planning by avoiding addition processing steps such as co-registration of images which is prone to errors. The patient may also benefit from a significant saving of examination time. Problems associated with dedicated MRA scan, as mentioned above, may be overcome. Examples may comprise one or more of the following features:
High-resolution T2 weighted scan in which the blood vessels are black.
Software algorithm(s) to extract the vascular information from the MRI image.
Visualize the angiogram in 3D.
Add the anatomical target area to the 3D view for pre-surgical planning.
Distinguish arterial and venous flow.

The MR data in the examples describe herein are recorded at 3 Tesla using a 3D T2-weighted turbo-spin echo sequence. The resolution at acquisition is 0.7 mm isotropic and the reconstructed data have a 0.35 mm isotropic resolution.

The features of this sequence may include one or more of the following: TR/TE: 3000/204 MS
Sagittal 543 slices, over contiguous
Acquisition resolution 0.7 mm isotropic, matrix: 356x329
Reconstruction resolution 0.35 mm isotropic
3D multi echo sequence (TSE)
8 or 32 channel head-coil
SENSE reduce scan time, shorter echo train length (ETL)
P reduction 1.4
S reduction 2
TSE
Linear
Turbo direction Y
Single shot
Echoes 162
Ultra short
3D non-selective
ES/shot: 6.2/950 ms
Half scan
Factor Y: 0.675
Factor Z: 1
Total scan-time is 6:50 minutes.

The contrast may depend on the chosen TR and TE, no additional pulse, e.g DRIVE, is used in some examples. The echo-train may be short in order to minimize blurring of the image. Length of the echo-train is controlled by both SENSE p-reduction and the halfscan factor y. It may be beneficial if the SENSE p-reduction should is low. Furthermore, the TE is controlled by the halfscan factor y. Current settings are a delicate comprise of the above considerations: optimal SNR in center of image, echo-train length and TE. Water-fat shift settings are not relevant, since TSE ultra-short is used, the maximum bandwidth setting always applies. Currently no fat suppression is used.

Since the voxels are isotropic the image can be viewed form the 3 principle directions without any additional processing or reformatting. This isotropic property is important for the anatomical analysis including the vessel detection. The image shows grey and white matter in different grey values. The cerebrospinal fluid is bright. Note that all brain vessels are black, visible as the black dots in the images.

As mentioned above, the vessel are black in the T2-weighted scan. A more detailed analysis shows that the blood vessels, both arteries and veins, are black as shown in Fig. 2 below.

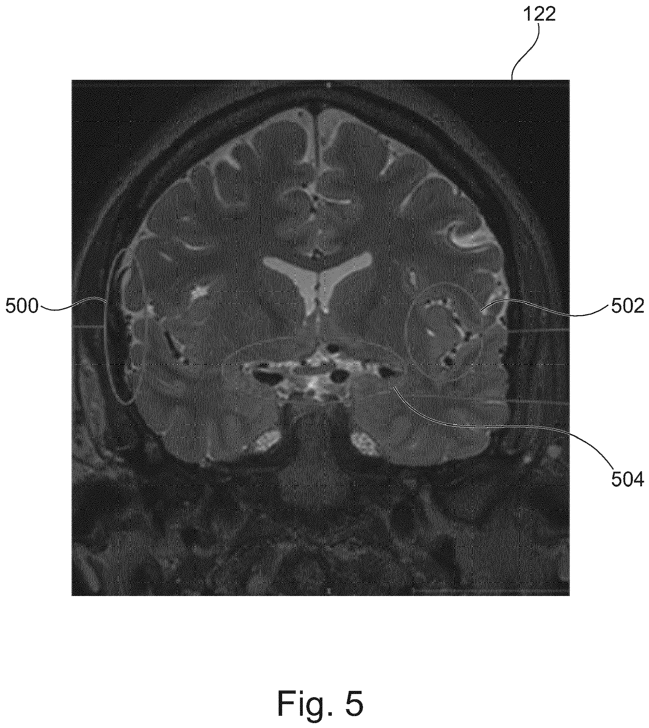

Fig. 5 shows an example of a dark blood T2 weighted magnetic resonance image 122. In this image the vascular system is clearly visible. There are cortical veins 500 which are visible as well as arteries 502. Arteries in the circle of Willis 504 are also visible.

The detailed analysis also showed that all arteries and veins are present. Vessels with a diameter in the order of the image resolution (0.35 mm) can be detected. For many clinical applications this more than sufficient.

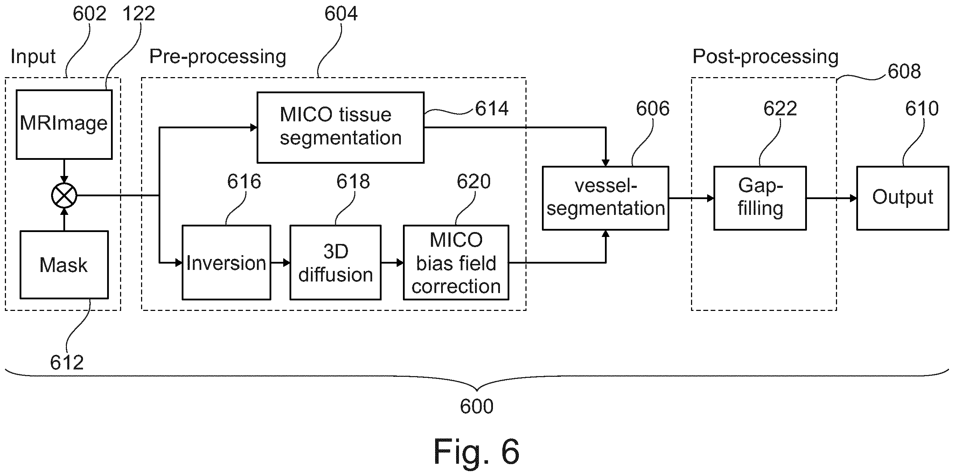

Fig. 6 shows an example of an automated blood vessel segmentation algorithm 600. This algorithm has an input portion 602 which feeds a preprocessing portion 604. The preprocessing portion 604 is then input into a vessel segmentation 606 algorithm portion. After the vessel segmentation 606 the segmentation is further processed by a post-processing portion 608. After the post-processing 608 is finished the algorithm outputs 610 the final segmentation. The input 602 takes the dark blood t2 weighted magnetic resonance imaging data 122 as input and also calculates a segmentation that provides a binary brain mask 612. These are then multiplied together to provide the brain tissue image. The brain tissue image is then processed on two different tracks in the preprocessing portion 604. There is a MICO tissue segmentation 614 on one track. In another track there is an inversion 616, a 3D diffusion 618 and then a MICO bias field correction 620 that is performed. The data from these two tracks is then input into the vessel segmentation 606. In the post-processing portion a gap-filling 622 operation is performed and then is finally output 610 as the magnetic resonance angiogram 124. Details for these steps are given in greater detail below.

The segmentation algorithm of fig. 6 implemented in three steps. The original head images are processed using SPM12, a well-known functional magnetic resonance imaging (fMRI) tool. The SPM12 segmentation tool provides images corresponding to the CSF, grey matter and white matter.

A binary mask of the brain is obtained by applying a binary OR operation on the segmented images from SPM12. An image hole filling operation is applied to the mask.

Finally, the brain tissue image is derived by multiplying the original images with the brain tissue binary mask.

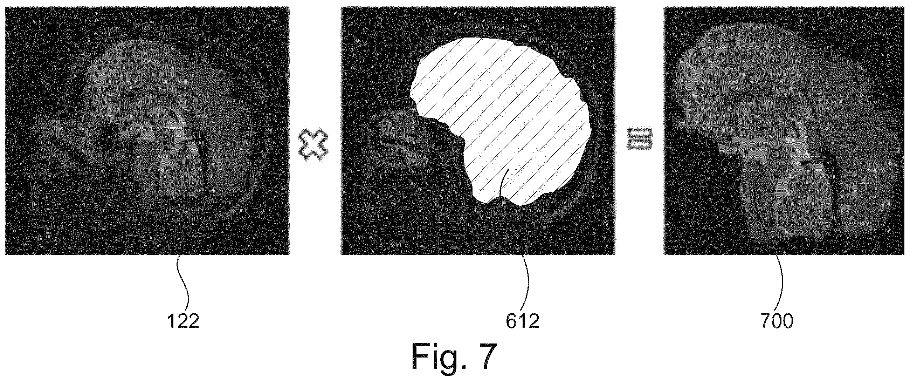

Fig. 7 illustrates the construction of the brain tissue image 700. Fig. 7 shows a dark blood T2 weighted magnetic resonance imaging data 122 and its associated binary brain mask 612. The two 122 and 612 are multiplied together and the result is the brain tissue image 700.

In the pre-processing stage 604 This stage targets on contrast enhancement and consists of two branches running in parallel.

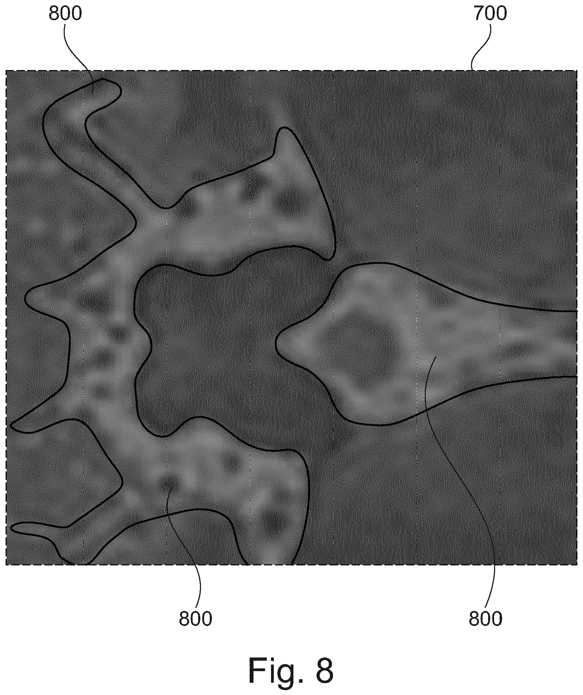

The upper branch 614 implements CSF tissue segmentation and generates a binary mask from the segmented brain tissue images. We apply the MICO tissue segmentation 614 in the original whole brain image. The CSF segmented image obtained from SPM12 often excludes the surface area of CSF region which is not the case for the MICO segmentation. An example of MICO segmentation is highlighted in Fig. 8 below. This generates the binary map BCSF used in the vessel segmentation.

Fig. 8 illustrates steps 614 in Fig. 6. Fig. 8 shows a portion of a brain tissue image 700 with a MICO CFS segmentation 800

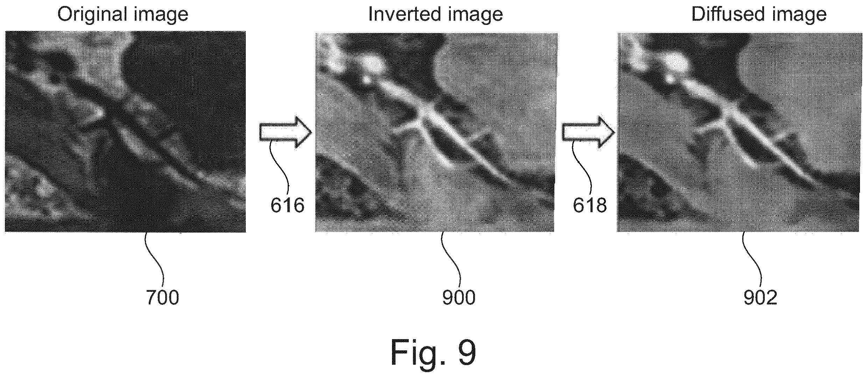

The lower branch 616, 618, 620 focuses on image enhancement. First step is to invert the image 616 (G-1 used in the vessel segmentation, see below), vessels are now bright. In order to reject possible false detections at the vessel segmentation stage a 3D-diffusion filter 618 is used. MICO bias field correction 620 equalizes the inhomogeneous intensity distributions in the images. This generates the image GH used in the vessel segmentation (see below). Exemplary images depicting performance enhancement are shown in Fig. 9 below.

Fig. 9 illustrates steps 616 and 618 of Fig. 6. In Fig. 9 the original brain tissue image 700 is depicted. The inversion 616 is then performed to construct an inverted brain tissue image 900. Then, the operations in 618 are performed and a three-dimensional diffusion filter is applied to the inverted image 900 to provide the diffused image or diffusion filter brain tissue image 902.

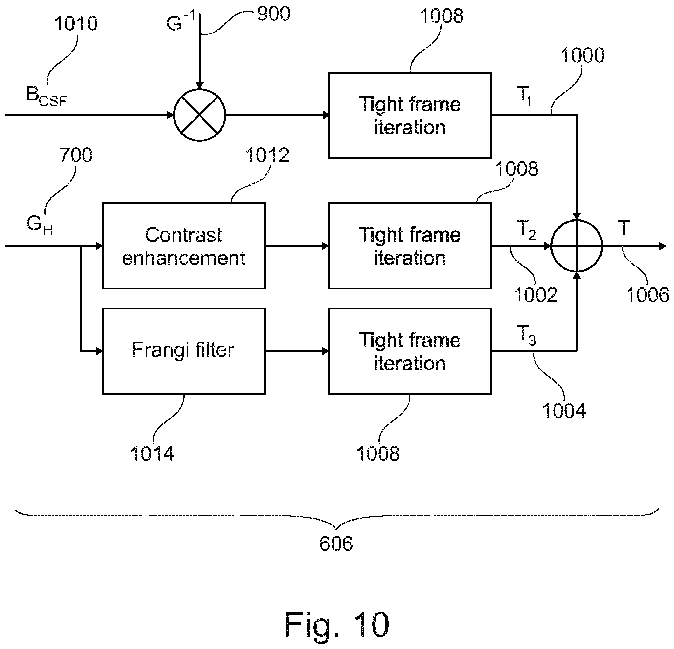

Fig. 10 illustrates an implementation of the vessel segmentation 606 illustrated in Fig. 6. BCSF, GH and G-1 are generated in the preprocessing as described above. The blood vessel segmentation 1006 is produced by a combination of three different binary vessel maps 1000, 1002, 1004. Each of these binary vessel maps 1000, 1002, 1004 are produced separately using a gradient-based segmentation algorithm 1008, in this case the tight frame iteration segmentation algorithm.

The binary vessel map 1000 or T₁ represents a first branch. This is done by multiplying the inverted brain tissue image 900 by a cerebrospinal fluid binary map 1010.

For the branches for the binary blood vessel map T₂ or the binary vessel map 1004 also known as T₃, the brain tissue image 700 is used. In the 1002 or T₂ branch a contrast enhancement filter 1012 is first applied before the gradient-based segmentation algorithm 1008 is applied. In the T₃ or 1004 branch, a Frangi filter 1014 is applied before the gradient-based segmentation algorithm 1008.

In Fig. 10, the structure is divided into two major parts: a branch T₁ and a combination of branches T₂ and T₃. These two parts are in correspondence with the essential assumption that the vessels lay either in the CSF region or nearby. The tight frame iteration is an image gradient-based algorithm, it denoises the image based on a complex wavelet transform.

The branch T₁, computation is done in the CSF region independently, in order to detect the smaller vessel structure that lay in the CSF region. First the binary map BCSF is multiplied with the inverted image G-1 before the tight frame iteration is applied.

The combination of branches T₂ and T₃ is dedicated to recover the relative bigger vessel structures that are located nearby the CSF regions. The computation is applied to the whole brain area (GH) Specifically, branches T₂ and T₃ detect respectively the bigger junction and the tubular structure.

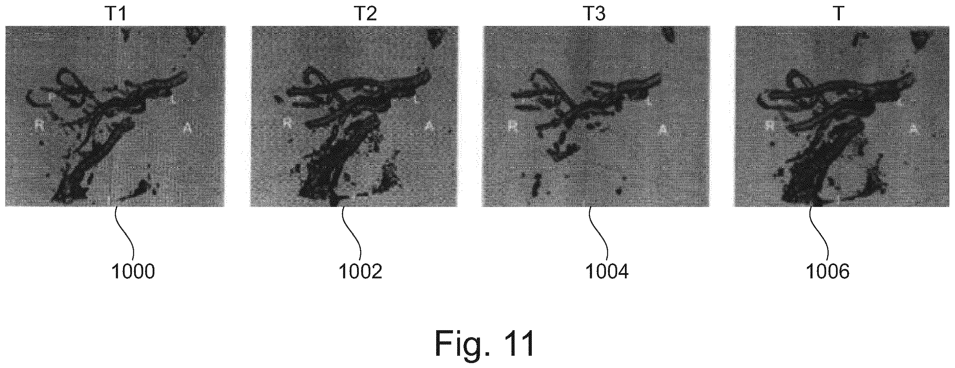

Fig. 11 shows the binary vessel map 1000 or T₁, the binary vessel map 1002 or T₂ and the binary vessel map 1004 or T₃. Fig. 11 also shows the blood vessel segmentation 1006 which is a combination of all three 1000, 1002, 1004.

All post-processing 608 is performed on the 3D binary mask (T) generated in the vessel segmentation

The hole-filling algorithm detects and fills the holes inside the blood vessels. These holes are caused by the gradient based tight frame iteration. In particular for the larger vessels, the algorithm will only detect the edge of the vessel and the inner part, which has small gradient changes will not be detected. For the filling algorithm, we assume that the intensity of the holes should be larger than that of the vessel edge in the inverted image G-1. Therefore, we perform threshold detection in the inverted image and when the threshold is higher than the average of the vessel edge, this will be a hole that will be filled in the binary maps T.

The gap-filling algorithm begins with skeletonization of the hole-filled images and adopts the definition of tokens. There are three types of tokens identified for the skeletonized network. The tokens are used to determine the gap position in the skeletonized network. Examples may use an iterative gap-filling algorithm, which takes a set of tokens as a starting point and iteratively propagates along the vessels structure with local vessel segmentation. Specifically, by using the branch T₃ in vessel segmentation, the vessel structure grows with three pixels within each iteration. Three pixels as an incremental step may be chosen in order to prevent possible artifacts, e.g. connecting arteries with veins. The proposed gap-filling algorithm may also removes the small particle noise. In addition, the gap filling algorithm is computationally efficient.

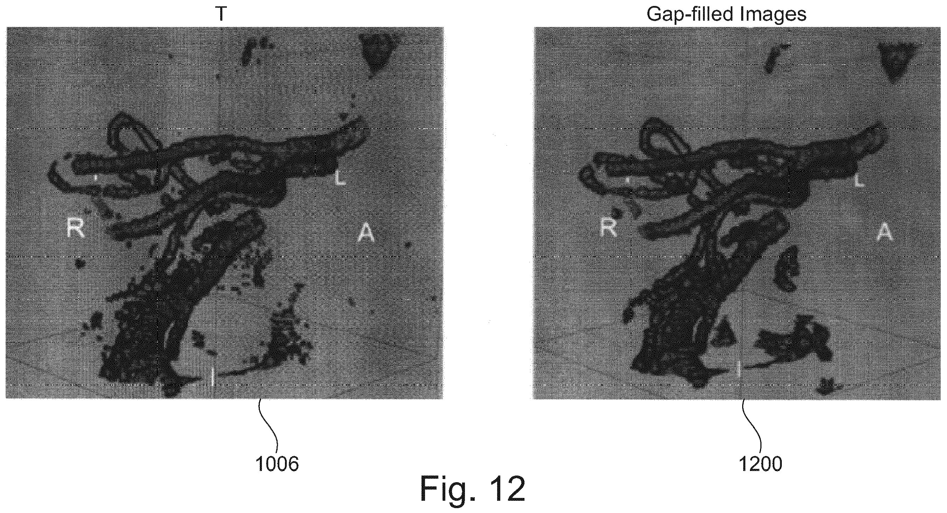

Fig. 12 shows a further view of the blood vessel segmentation 1006 depicted in Fig. 11. It also depicts a gap-filled image 1200 constructed from the blood vessel segmentation 1006.

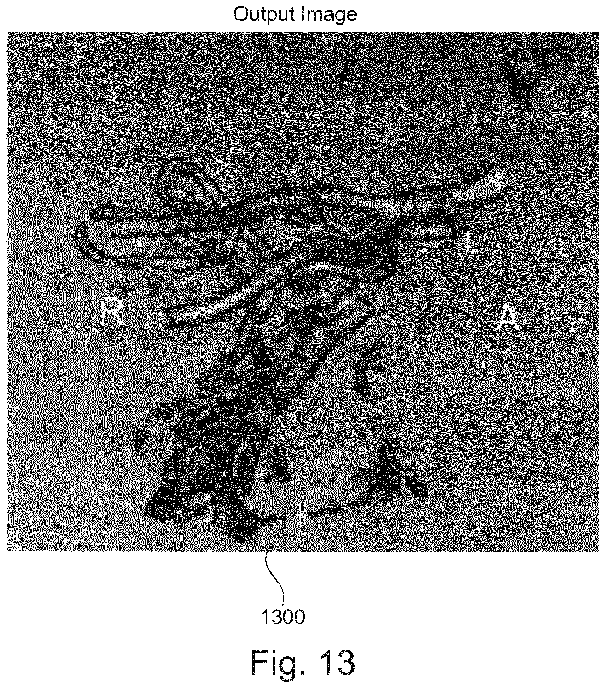

In order to improve the visibility, the binary image was multiplied by the diffused and scaled (power of 6) image. The final output image 1300 is shown in Fig 13.

The target for DBS in patients with Parkinson's disease is the subthalamic nucleus (STN). This structure is indeed located deep in the brain and can be identified on the anatomical images. Combining the angiogram and the anatomical target in a 3D view will enable the surgeon to determine a save path for the needle from the entry point on the skull to the STN, avoid all major vessel and/or beware of the vessels which are close to the needle trajectory.

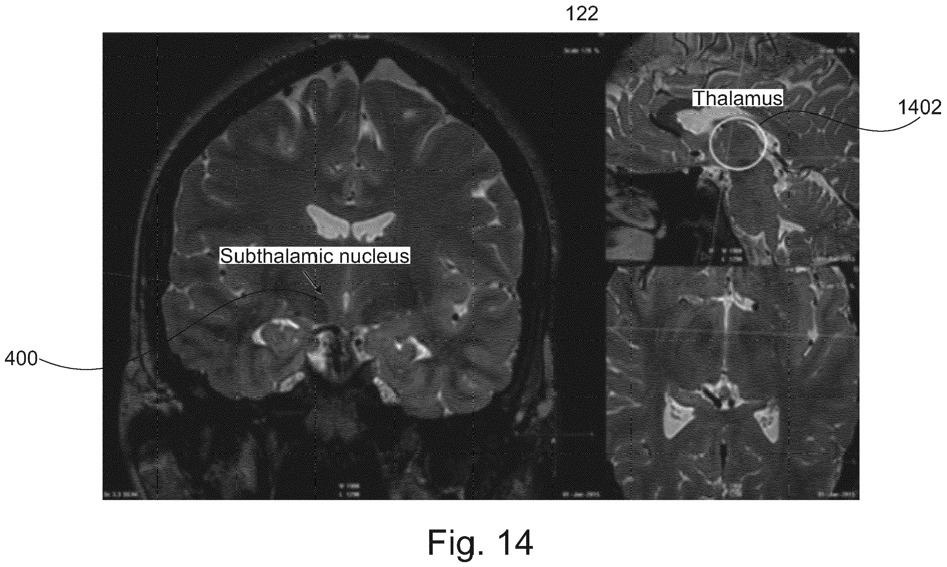

Fig. 14 shows an example of a dark blood T2 weighted magnetic resonance imaging data 122 of a subject's brain. The subthalamic nucleus 1400 close to the base of the skull and the thalamus 1402 are visible.

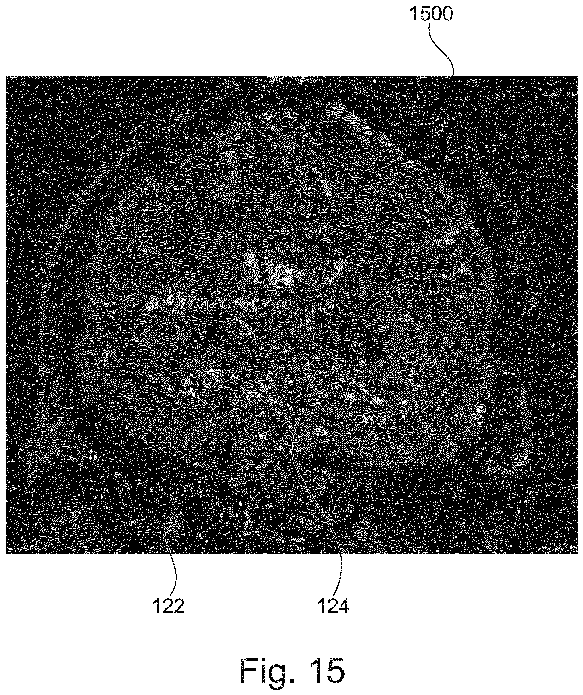

The anatomical target area and the angiogram, both derived from the same scan will be shown in a 3D view for the pre-surgical planning. Fig. 15 below shows an overlay of the angiogram in a coronal view on the anatomical data, note this image is just a graphical representation and note an exact derivative of the data.

Fig. 15 shows a further view of the dark blood T2 weighted magnetic resonance imaging data 122 depicted on the left side of Fig. 14. In Fig. 15 a superposition of the magnetic resonance angiogram is shown as being over the dark blood T2 weighted magnetic resonance imaging data 1500. The image 1500 is therefore a composite or combination of the magnetic resonance angiogram and the dark blood T2 weighted magnetic resonance imaging data. This image would be useful for planning a surgical intervention.

If needed, it may be possible to separate arterial and venous flow. The arterial flow can be followed starting from the carotid arteries using vessel tracking algorithms. Note that arteries and veins are connected true the capillary bed, these vessels may be too small to see in the angiogram, therefore, vessel tracking of the arteries could possibly never enter the venous system.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

- 100: medical system
- 102: computer
- 104: computational system
- 106: hardware interface
- 108: user interface
- 110: memory
- 120: machine executable instructions
- 122: dark blood T2 weighted magnetic resonance imaging data
- 124: magnetic resonance angiogram
- 200: receive dark blood T2 weighted magnetic resonance imaging data descriptive of a brain region of a subject
- 202: segment the dark blood T2 weighted magnetic resonance imaging data for blood vessels to provide a magnetic resonance angiogram of the brain region
- 300: medical system
- 302: magnetic resonance imaging system
- 304: magnet
- 306: bore of magnet
- 308: imaging zone
- 309: region of interest
- 310: magnetic field gradient coils
- 312: magnetic field gradient coil power supply
- 314: radio-frequency coil
- 316: transceiver
- 318: subject
- 320: subject support
- 330: pulse sequence commands
- 332: k-space data
- 334: rendering of dark blood T2 weighted magnetic resonance imaging data and magnetic resonance angiogram
- 336: render a therapy path (chosen using user interface)
- 400: acquire the k-space data by controlling the magnetic resonance imaging system with the pulse sequence commands
- 402: reconstruct the dark blood T2 weighted magnetic resonance imaging data from the k-space data
- 404: render the dark blood T2 weighted magnetic resonance imaging data and the magnetic resonance angiogram three dimensionally to provide a therapy planning user interface
- 500: corticl vein
- 502: arteries
- 504: arteries circle of Willis
- 600: automated blood vessel segmentation algorithm
- 602: input portion
- 604: pre-processing portion
- 606: vessel segmentation
- 608: post procession portion
- 610: output
- 612: binary brain mask
- 614: MICO tissue segmentation
- 616: Inversion
- 618: 3D-diffusion
- 620: MICO bias filed correction
- 622: Gap-filling
- 700: brain tissue image
- 800: MICO CSF segmentation
- 900: inverted brain tissue image
- 902: diffusion filtered brain tissue image
- 1000: binary vessel map
- 1002: binary vessel map
- 1004: binary vessel map
- 1006: blood vessel segmentation
- 1008: gradient based segmenation algorithm
- 1010: CSF binary map
- 1012: contrast enhancement filter
- 1014: Frangi filter
- 1200: gap filled image
- 1300: output image
- 1400: subthalamic nucleus
- 1402: thalamus
- 1500: magnetice resonance angiogram superimposed on dark blood T2 weighted magnetic resonance imaging data

## Claims

1. A medical system (100, 300) comprising:
- a memory (110) storing machine executable instructions (120), and
- a computational system (104), wherein execution of the machine executable instructions causes the computational system to:
- receive (200) dark blood T2 weighted magnetic resonance imaging data (122) descriptive of a brain region of a subject; and
- segment (202) the dark blood T2 weighted magnetic resonance imaging data for blood vessels to provide a magnetic resonance angiogram (124) of the brain region.

2. The medical system of claim 1, wherein execution of the machine executable instructions causes the computational system to segment the blood vessels in the dark blood T2 weighted magnetic resonance imaging data with the steps comprising:
- obtaining a binary brain mask (612) for the dark blood T2 weighted magnetic resonance imaging data by inputting the dark blood T2 weighted magnetic resonance imaging data into a brain segmentation algorithm;
- obtaining a brain tissue image (700) by multiplying the dark blood T2 weighted magnetic resonance imaging data with the binary brain mask; and
- providing a blood vessel segmentation by segmenting the brain tissue image for blood vessels; and
- providing the blood vessel segmentation as the magnetic resonance angiogram.

3. The medical system of claim 2, wherein execution of the machine executable instructions further causes the computational system to provide the blood vessel segmentation by summing blood vessel segmentations of multiple images (1000, 1002, 1004) derived from the brain tissue image.

4. The medical system of claim 3, wherein the segmentation of the dark blood T2 weighted magnetic resonance imaging data comprises a cerebral spinal fluid region segmentation, wherein execution of the machine executable instructions further causes the computational system to:
- calculate an inverted brain tissue image (900) by inverting the brain tissue image;
- calculate an inverted cerebral spinal fluid region using the cerebral spinal fluid region segmentation and an inversion of the brain tissue image, wherein the multiple images derived from the brain tissue image comprise the inverted cerebral spinal fluid region.

5. The medical system of claim 4, wherein execution of the machine executable instructions further causes the computational system to:
- calculate (616) a diffusion filtered brain tissue image (902) by applying a three-dimensional diffusion filter to the inverted brain tissue image;
- calculate (1012) a contrast enhanced brain tissue image by applying a contrast enhancement filter to the diffusion filtered brain tissue image, wherein the multiple images derived from the brain tissue image comprise the contrast enhanced brain tissue image; and
- calculate (1014) a Frangi filtered brain tissue image by applying a Frangi filter to the diffusion filtered brain tissue image, wherein the multiple images derived from the brain tissue image comprise the Frangi filtered brain tissue image.

6. The medical system of any one of claims 3, 4, or 5, wherein the segmentation of the multiple images is performed using a gradient based segmentation.

7. The medical system of any one of claims 3 through 6, wherein execution of the machine executable instructions further causes the computational system to construct the magnetic resonance angiogram by applying (622) a gap filling algorithm to the combined segmentations of multiple images.

8. The medical system of any one of the preceding claims, wherein execution of the machine executable instructions further causes the computational system to identify arterial and venous flow in the magnetic resonance angiogram.

9. The medical system of any one of the preceding claims, wherein execution of the machine executable instructions further causes the computational system to render (404) the dark blood T2 weighted magnetic resonance imaging data and the magnetic resonance angiogram three dimensionally to provide a therapy planning user interface, wherein the magnetic resonance angiogram is superimposed on the dark blood T2 weighted magnetic resonance imaging data during the rendering, and wherein the therapy planning system is further configured to render a therapy path in the therapy planning user interface.

10. The medical system of claim 9, wherein the therapy path is any one of the following, a radiotherapy path, a biopsy needle insertion path, a deep brain stimulation electrode insertion path, and a surgical path.

11. The medical system of any one of the preceding claims, wherein the medical system further comprises a magnetic resonance imaging system (302) controlled by the computational system, wherein the memory further stores pulse sequence commands configured (330) for acquiring k-space data (332) according to a dark blood T2 weighted magnetic resonance imaging protocol, wherein execution of the machine executable instructions further causes the computational system to:
- acquire (400) the k-space data by controlling the magnetic resonance imaging system with the pulse sequence commands; and
- reconstruct (402) the dark blood T2 weighted magnetic resonance imaging data from the k-space data.

12. The medical system of claim 11, wherein the dark blood T2-weighted magnetic resonance imaging protocol is a 3D T2-weighted turbo-spin echo magnetic resonance imaging protocol.

13. The medical system of any one of the preceding claims, wherein the dark blood T2 weighted magnetic resonance imaging data has a resolution of 0.5 mm or lower, wherein the dark blood T2 weighted magnetic resonance imaging data preferably has a resolution of 0.4 mm or lower, and wherein the dark blood T2 weighted magnetic resonance imaging data more preferably has a resolution of 0.35 mm or lower.

14. A computer program comprising machine executable instructions (120) for execution by a computational system (104), wherein execution of the machine executable instructions causes the computational system to:
- receive (200) dark blood T2 weighted magnetic resonance imaging data (122) descriptive of a brain region of a subject; and
- segment (202) the dark blood T2 weighted magnetic resonance imaging data for blood vessels to provide a magnetic resonance angiogram (124) of the brain region.

15. A method of medical imaging, wherein the method comprises:
- receiving (200) dark blood T2 weighted magnetic resonance imaging data descriptive of a brain region of a subject; and
- segmenting (202) the dark blood T2 weighted magnetic resonance imaging data for blood vessels to provide a magnetic resonance angiogram of the brain region.
